Europäisches Patentamt

European Patent Office

Office européen des brevets

(11) Publication number: **0 425 968 A2**

## EUROPEAN PATENT APPLICATION

(21) Application number: 90120244.0

(22) Date of filing: 22.10.90

(51) Int. Cl.⁵: **A61K 9/70, A61L 15/16, A61M 35/00**

(30) Priority: 23.10.89 US 425079

(43) Date of publication of application:
08.05.91 Bulletin 91/19

(84) Designated Contracting States:
AT BE CH DE DK ES FR GB GR IT LI LU NL SE

(71) Applicant: G.D. Searle & Co.
P.O. Box 5110
Chicago Illinois 60680(US)

(72) Inventor: Farhadieh, Bahram
965 Sunrise Road
Libertyville, Illinois 60048(US)
Inventor: Vallner, Joseph
1211 Lisa Court
Los Altos, California 94022(US)
Inventor: Berger, Hana
21 Nutmeg Drive
New Milford, Connecticut 06776(US)

(74) Representative: Beil, Hans Christoph, Dr. et al
Beil, Wolff und Beil Rechtsanwälte
Adelonstrasse 58
W-6230 Frankfurt am Main 80(DE)

(54) Novel multiple layer transdermal drug administration system.

(57) A multi-layer patch for the transdermal delivery of pharmaceutical drugs. The patch is characterized by having multiple masses of silicone elastomer in which the active drug and a percutaneous absorption enhancer are homogeneously dispersed throughout. The patch is especially well suited to delivering the beta₂ adrenergic agonist drug albuterol.

EP 0 425 968 A2

## NOVEL MULTIPLE LAYER TRANSDERMAL DRUG ADMINISTRATION SYSTEM

BACKGROUND OF THE INVENTION

The present invention relates to a multi-layered transdermal patch for the administration of drugs percutaneously. In particular, the invention is useful for the administration of the drug albuterol, a $\beta_2$ adrenergic agonist, which is useful, among other things, in the treatment of asthma by virtue of its action of inducing bronchodilation.

The practicality of administering a given drug percutaneously on a continuous basis depends upon the concentration of drug in the blood that is required to provide the desired pharmacologic effect, the degree to which the skin is permeable to the drug, and the amount of skin surface area that is available for administration. The available skin surface area, while theoretically being unlimited, is, for practical reasons, typically confined to a range of from about five square centimeters to about 100 square centimeters. With the available area fixed within this range, the matter then narrows as to whether sufficient drug will pass through that much area to provide the desired therapy. If it will, then it is simple to effectively administer the drug percutaneously. If, however, the inherent permeability of the skin to the drug is so high or so low that too much or too little drug will pass through that area of skin, then the rate of administration of the drug to the skin must be controlled or the permeability of the skin to the drug must be increased, as the case may be, to make percutaneous administration practical. The present invention involves an approach in which the active drug component's percutaneous administration is enhanced by the presence of a percutaneous absorption enhancer, also known as a diffusion enhancer.

Systemically active drugs are conventionally administered either orally or by injection, with the primary objective of either mode being to achieve a given desired blood level of drug in circulation over a period of time. However, these prior conventional methods possess certain shortcomings resulting in the failure to obtain these goals. For example, the oral route is inadequate for several reasons, even though the drug is administered at periodic intervals according to a well defined schedule. The rate of absorption of drug through the gastrointestinal tract is affected by both the contents of the tract and the passage of time as a drug travels through the small intestine. Therefore, such variables as whether the drug is administered before or after eating and the type and quantity of food eaten (for example, high or low fat content), or whether administered before or after a bowel movement, affect the rate of absorption of the drug which takes place in the small intestine. Additionally, the time of passage of drug through the small intestine is affected by the rate of peristaltic contraction, adding further uncertainty. Also important is the rate of circulation of blood to the small intestine and the fact that many drugs administered by this route are rendered inactive by gastric acid and digestive enzymes of the gastrointestinal tract or liver where the drug can be metabolized to an inactive product by that organ. These factors make it difficult to achieve a desired time course of concentration of the drug in the blood. The most inevitable result of oral administration of drugs through the gastrointestinal tract is that the level of drug in circulation surges to a peak level at the time the drug is administered, followed by a decline in concentration in the blood and body compartments. Thus, a plot of drug in circulation after administration of several tablets a day will have the appearance of a series of peaks which may surpass the toxic threshold of the drug, and valleys which may fall below the critical point needed to achieve the desired pharmacologic or therapeutic effect.

The administration of drugs by injection can likewise entail certain disadvantages. For example, very strict asepsis must be maintained in order to avoid infection of the blood, the vascular system or the heart. Drug administration by poor intravenous injection technique may result in perivascular injection when that was not intended; the typical result of injection into the blood is a sudden rise in the blood concentration of the drug followed by an uncontrollable decline. Additionally, administration of drugs by injection is inconvenient and painful. Other dosage forms for systemic administration or drugs, such as rectal suppositories and sublingual lozenges, also produce non-uniform levels of the therapeutic agent in circulation. These dosage forms require great patient cooperation and have low patient acceptability, resulting in decreased patient compliance with a prescribed drug regimen, which is the most common failure of drug therapy.

To avoid the problems discussed above, a new branch of drug delivery has developed in which systemically active drugs are administered through the intact skin. Uncertainties of administration through the gastrointestinal tract and the inconvenience of administration by injection are eliminated. Since a high concentration of drug never enters the body, problems with pulse entry are overcome. Despite these advantages of administering systemically active drugs through the skin, many problems exist with prior art devices designed for this purpose. Many such devices do not provide continuous administration or continuous delivery rate. Also, many such devices have limited application to a relatively narrow group of therapeutic drugs. Frequently, new application systems must be designed for drugs which are simply incompatible with prior art application systems.

The present invention seeks to overcome prior problems with continuous administration and delivery rate in general, and has been found to work particularly well with adrenergic agonists, and especially well with albuterol, a selective $\beta_2$ adrenergic agonist. Another object of this invention is to provide a device for the administration of albuterol in a reliable and easily applied device for continuously administering the drug in controlled quantities through intact skin or mucosa. Another object of this invention is to provide for such a device that will cause little, if any, dermal irritation. Another object of this invention is to provide a device that will be especially useful and acceptable in pediatric patients and geriatric patients. A further object of this invention is to provide a device not relying on the use of an adhesive. Yet another object of this invention is to provide a device which will provide continuous dosing of the patient over a 24-hour period.

The patch of the present invention features 100% bioavailability, good margin of safety in pediatric or geriatric patients, ease of administration, and little or no dermal or mucosal irritation.

Transdermal albuterol would be useful for actual asthma therapy, rather than merely prophylaxis. It would be useful in pediatric age groups and geriatric populations, both of which require simple to administer regimens that do not rely on responsibility or memory of the patient to comply with 2, 3 or 4 time daily dosage administration, as is often needed with conventional tablets or capsules. Transdermal albuterol therapy would be useful after treatment of an acute asthma attack to prevent exacerbation of such an attack. Clinically, it would also be useful either as a substitute for IV therapy or as an improvement over oral therapy.

In addition to being convenient, transdermal albuterol therapy would have a significant margin of safety. Significantly, an on-going therapy, say with sustained release oral formulations, could be interrupted if the average plasma level were too high. Once the patient were stabilized at a lower plasma level, the transdermal albuterol patch would be available to maintain consistent plasma levels at a more desirable lower level.

Additionally, albuterol can be used as a tocolytic (obstetric) agent. Preterm labor occurs in approximately 10% of pregnancies. Commonly, beta-mimetic agents are indicated for preterm labor. Albuterol is currently used for preterm labor, with the plasma levels needed for uterine relaxation being 8 to 33 nanograms per milliliter. Such levels are within the range delivered by the present invention. The albuterol patch has the potential advantage of safety over the IV route and the further advantage of more even dosing over the oral route during the sensitive and critical period during which labor occurs. Such use can be adjunctive with bed rest, IV and oral agents, or could be primary therapy as a substitution for intravenous beta-mimetic agents.

Additionally, a transdermal albuterol patch may even find usage as emergency therapy for the treatment of urticaria (hives).

Albuterol's usefulness as a bronchodilator is not limited to the treatment of asthma. Albuterol can also be used as a bronchodilator in treatment of bronchitis, chronic obstructive pulmonary disease or other obstructive pulmonary diseases.

## DESCRIPTION OF THE DRAWINGS

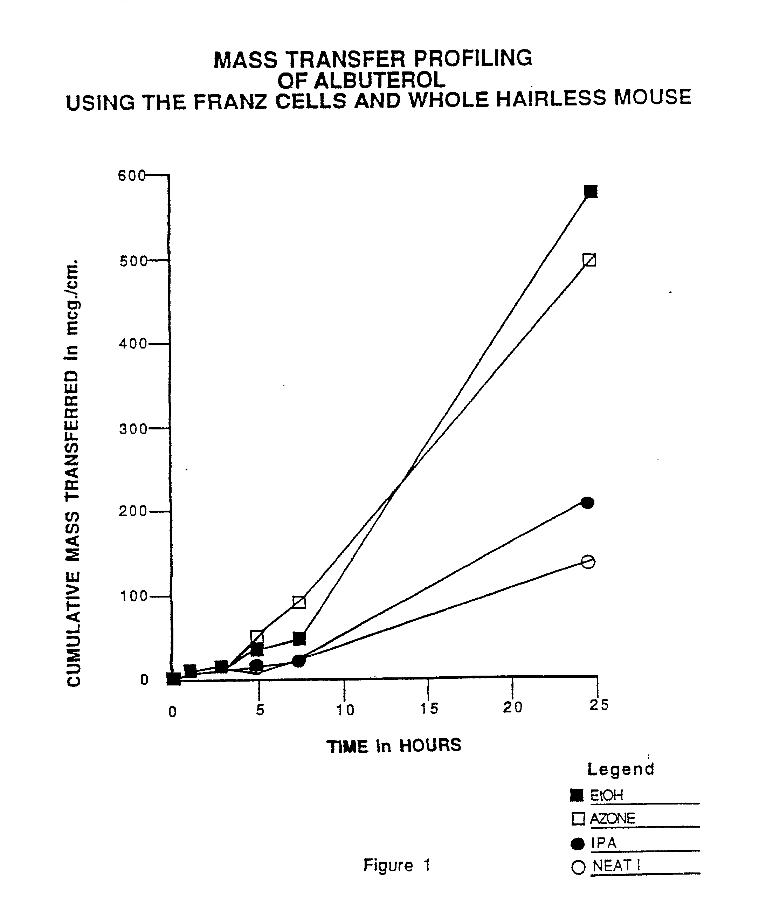

Figure 1 is a plot of albuterol transfer versus time, showing how various adjuvant agents affect the flux of albuterol. It can be seen that ethanol was most effective against prior art agents such as Azone® and isopropyl alcohol (IPA).

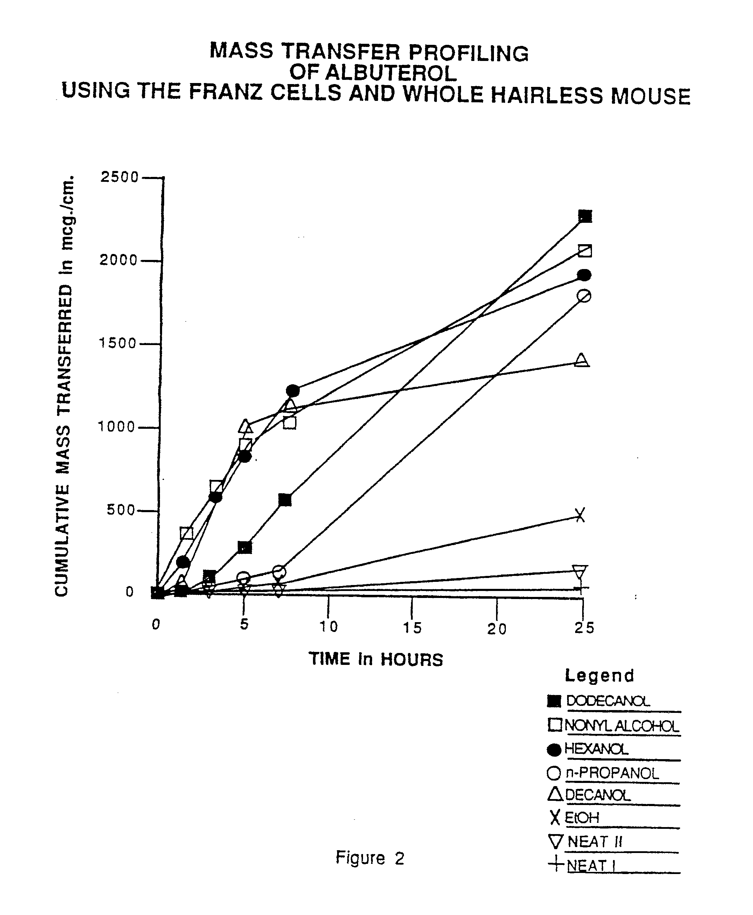

Figure 2 is the same type of plot, except that all agents tested are normal hydrocarbon alcohols. It can be seen that agent effect on albuterol flux increases as chain length of the alcohol increases.

## SUMMARY OF THE INVENTION

The most preferred embodiment of this invention resides in a laminated multi-layer patch for the transdermal administration of a drug comprising at least two elastomeric matrices of predetermined thickness and area; an active drug ingredient dispersed throughout at least one of the matrices; and a diffusion enhancer dispersed throughout at least one of the matrices. Additionally, a suitable plasticizer and/or a solubilizing agent for the active ingredient can conveniently be added to the patch.

Suitable matrix materials comprise the following polymers:

Polyethylene,

Polypropylene,
Polyethylene terphthalate,
Polyvinylidene fluoride,
Polymethyl methacrylate,
Polyurathane-polyamide copolymers,
Poly(2-hydroxyethyl methacrylate)
(HEMA-hydrogel),
Polyalkyl acrylate esters (bioadhesive polyemer),
Polyisobutylene (Bioadhesive polymer),
polydimethylsilicone with resin (Bioadhesive polymer) or
Silicone elastomers.

The patch preferably utilizes a silicone elastomer as the matrices. Silicone elastomers have alternating silicon and oxygen atoms for a backbone. Double bonds are absent in such a backbone, and therefore, the numerous forms of stereoisomers ordinarily found in unsaturated hydrocarbon rubbers do not have counterparts in the silicone rubbers. An especially useful silicone elastomer is Silastomer™ X7-3058, available from Dow Corning, Inc.

In the most preferred embodiment of the invention, the active drug ingredient is albuterol, most preferably as the free base.

Diffussion enhancers are suitably chosen from the group comprising:
Decymethyl Sulphoxides,
Hexylmethyl Sulphoxide,
Trimethyl phosphine oxide,
N,N Dimethyl-m-toluamide,
Tetrahydrofurfuryl alcohol,
Dimethyl acetamide,
Propylene glycol,
n-methyl-2-pyrrolidone,
2-pyrolidone,
1-ethyl-2-pyrolidone,
Sodium lauryl phosphate,
Triethanol amine lauryl phosphate,
Poloxomer 231,
Polyoxyethylene 4 lauryl ether,
Poloxomer 182,
Urea,
Isopropyl myristate,
Isopropyl palmitate,
Butyrolactone,
Vanillin,
Stearyl alcohol or
the normal hydrocarbon alcohols.

Preferred diffusion enhancers are normal hydrocarbon alcohols, with the most preferable diffusion enhancer being n-dodecanol, dispersed throughout at least one of the elastomeric matrices.

## DETAILED DESCRIPTION OF THE INVENTION

In accordance with this invention, there is provided a patch suitable, by virtue of the rate controlling materials employed therein, for the predetermined controlled administration of drug to the skin or mucosa of a mammal over a period of time. To use the patch of the invention, it is applied to the patient's skin or mucosa and should be in firm contact therewith so as to form a tight seal. Flow of drug from the patch is metered through the material of the patch in accordance with the laws of diffusion, as hereinafter discussed, at a predetermined rate. In operation, drug molecules are continuously removed from the patch, migrating throughout it to the skin or mucosa of the patient where the drug is absorbed and enters circulation through the capillary network.

The rate of passage or permeation of drug through the material of the patch is determined by diffusive flux of drug molecules as is the case where the rate controlling material is of a solid nature in which the drug molecules can dissolve in and flow through to a direction of lower chemical potential. For this drug transfer mechanism, the release rate can be controlled in accordance with Fick's First Law, depending on the particular design by selection of dependent variables such as the diffusivity and solubility of the drug in the diffusive medium and by the thickness of the material of the patch. The mechanism of action of the diffusion enhancers herein may be to increase diffusivity of active ingredient through the matrix, or to increase percutaneous absorbtion through the skin or mucous tissue, or both, and both activities shall be understood to attach to the term "diffusion enhancer" as used herein.

Preferred elastomers useful in making the patch are the organopolysiloxane rubbers, commonly known as silicone rubbers. Suitable silicone rubbers are the conventional heat vulcanizable (curable) silicone rubbers and the room temperature vulcanizable silicone rubbers. Room temperature vulcanizable silicone rubbers will require the use of a curing agent or catalyst. The most especially preferred silicone rubber is SilastomerTM X7-3058, available from Dow Corning, Inc. Other room temperature vulcanizable silicone rubbers are also commercially available and are known to the art. A typical catalyst that will cure silicone rubber at room temperature is stannous 2-ethylhexoate, which can be present in a range of from 0.0625 to 0.5%, the most efficient amount being determinable by methods well known to those of ordinary skill in the art. Exemplary patents disclosing the preparation of silicone rubbers are U.S. Patent Nos. 2,541,137; 2,723,966; 2,863,846; 2,890,188; 2,927,907; 3,002,951 and 3,035,016. Elastomer can be present in an amount ranging from about 25 to 95 per cent, weight to weight. More preferably, it can be present from about 65 to 90 per cent, weight to weight.

A diffusion enhancer is used in the patch of the present invention, with most preferred diffusion enhancers being the normal hydrocarbon alcohols of one to twenty carbon atoms. As the chain length of the alcohol increases, the effectiveness of the diffusion enhancer increases up to a point. The most preferred diffusion enhancer is n-dodecanol. n-Dodecanol can be present in an amount ranging from about 2 to about 30 per cent, weight to weight. More preferably, it can be present in an amount ranging from about 3 to about 7 per cent.

Plasticizers are useful in increasing the plasticity of polymers. In a preferred embodiment of the present invention, a suitable plasticizer is desirable. Preferred plasticizers include diols, triols, and other polyols.

The most preferred plasticizer is glycerol. When albuterol is the active drug chosen for the patch of the present invention, albuterol can act as a self-plasticizer, due to its amine moiety.

In one preferred embodiment of the present invention, a solubilizing agent for the active ingredient can be desirable. Preferred solubilizing agents likewise include the normal hydrocarbon alcohols, with n-hexanol being the most preferred solubilizing agent (n-hexanol can also act as a useful plasticizer).

The most preferred patch of the present invention comprises Dow Silastomer TM X7-3058, 79.55% w/w, albuterol, 10.00% w/w, n-dodecanol, 5.00% w/w, glycerol, 2.50% w/w, and hexanol, 0.50%, w/w, with a suitable organotin catalyst, 0.50%, w/w (all percents are weight of ingredient in all layers to total weight of all layers in a multi-layer patch).

All materials used in the patch of the present invention are dispersed uniformly throughout the matrices created by use of the elastomer. The effective amount of active agent incorporated within a matrix layer to obtain the desired therapeutic effect will vary depending upon the desired dosage, the length of time the patch is to remain on the skin or the body mucosa and the area of the patch. Serum concentrations can be adjusted either by varying the albuterol concentration in the patch or by varying the patch size. Since the patch of this invention is designed to control drug administration for an extended period of time, ideally 24 hours or more, there is no critical upper limit on the amount of agent incorporated into the patch. The lower limit is determined by the fact that sufficient amounts of the agent must remain in the patch to maintain the desired dosage.

In order to achieve a therapeutic effect with albuterol in a human adult, the serum concentration of albuterol should be in the range of between about 2 to about 33 nanograms per milliliter, and most preferably from about 4 to about 8 nanograms per milliliter. 4 to 8 nanograms per milliliter is desirable for treating bronchoconstriction, and about 8 to 33 nanograms per milliliter is desirable for using albuterol as a tocolytic agent.

The effective rate of release of the active agent to the skin or mucosa can be in the range of from about 0.2 to 2.0 milligrams per square centimeter per day. A more preferred range would be from about 0.3 to about 0.85 milligrams per square centimeter per day. The exact amount will depend on the desired dosage as well as the condition to be treated. Those skilled in the art can readily determine the rate of permeation of active drug ingredient through the material or selected combinations of materials. Standard techniques are described in the Encyclopedia of Polymer Science and Technology, Volumes 5 and 9, pages 65 to 85 and 795 to 807, 1968; and the references cited therein, the disclosure of which is incorporated herein.

Albuterol can be present in an amount ranging from about 2 to about 30 per cent, weight to weight. More preferably, it can be present in an amount ranging from about 5 to about 25 per cent, and most preferably, in an amount ranging from about 6 to about 16 per cent, weight to weight.

Various occlusive or non occlusive, flexible or non-flexible backing members can be used in the patch in the present invention, if desired. Suitable backings would include cellophane, cellulose acetate, ethylcellulose, plasticized vinylacetate-vinylchloride copolymers, polyethylene terephthalate, nylon, polyethylene, polypropylene, polyvinylidenechloride, paper, cloth, or aluminum foil.

To prevent passage of the drug away from the exposed surface of the patch prior to use, the surface generally can be covered with a protective release film or foil such as waxed paper. Alternatively, the exposed rear surface of the backing member can be coated with a low-adhesion back side. To enhance stability of the active compounds, the patch usually is packaged between hermetically sealed polyethylene terephthalate films or aluminum foils under an inert atmosphere, such as gaseous nitrogen.

To use tne patch or the invention, it is applied to the the patient's skin. The patch should be in firm contact with the skin, preferably forming a tight seal therewith. Drug within the patch migrates through the patch to the skin by diffusion. When drug is in contact with the patient's skin, drug molecules which are continuously removed from the outer surface of the patch migrate through and are absorbed by the skin, entering the circulation through the capillary network. The patch can be applied to any area of the patient's skin, including the oral mucosa, for example, by application of the patch to the palate or the buccal mucosa. In addition, the patch of the invention can be used to administer drugs to other mucosa of the body, for example, it can be applied to the vaginal mucosa, the rectal mucosa, etc.

The following examples are merely illustrative of the present invention and should not be construed as limiting the scope of the invention in any way, as these examples and other equivalents thereof will become apparent to those skilled in the art in light of the present disclosure and accompanying claims.

## Example 1

Franz cell experiments . An eight week old male hairless mouse was sacrificed by spinal dislocation and a rectangular piece of abdominal skin was carefully lifted and separated from the adhering fatty tissue and visceral material. The skin tissue was mounted and clamped between the donor and the receptor compartments of a Franz cell with the epithelium facing the donor compartment. The temperature of the receptor was maintained by the external water bath set at 37° C, and the receptor solution was stirred with a magnetic stirrer. The receptor compartment was then charged with the normal saline solution, bathing the dermis of the skin tissue. Thus, the dermis was washed to remove the adhering cell debris. After two hours, the receptor solution was withdrawn and replaced with the fresh saline solution. Following this, a 1.38 square centimeter portion of the pad was cut and glued (355 Medical adhesive, Dow Corning, Midland, MI) on the adhesive foam (Fasson, Painville, OH). The delivery system containing the pad and an adhesive foam was then applied on the epidermal side of the skin. At the predetermined time intervals, 300 microliter samples of the receptor solution were removed from the sampling port, and replaced with the equivalent volume of normal saline solution. The samples were filtered, and quantitated by an HPLC method. Concentrations were converted into amounts, accounting for the dilution factor which was the volume of the receptor solution (7 ml). The amounts were then normalized for the area (1.38 cm$^2$) to calculate skin permeation rates (mg.cm$^{-2}$.day$^{-1}$) (Table I).

Pad residue analysis experiments: Pad residue analysis was conducted simultaneously with the pharmacokinetic experiments in monkeys.

Dissolution Experiments: A 8 square centimeter pad was cut and mounted on a holder of the Hansen's dissolution test apparatus, exposing 4 square centimeter area. The test apparatus was assembled and the test conducted using water as dissolution medium at 37° C. The dissolution medium was stirred at 50 rpm and five milliliter samples removed from the sampling port at the periodic intervals. These were then analyzed by an High Performance Liquid Chromatographic Method (HPLC). The chromatographic peaks were quantitated usig a peak height method to determine concentrations. These were then converted into amounts, accounting for the dilution factor which was volume of the dissolution medium (300 ml). The amounts were normalized for the area (4cm$^2$) to calculate the dissolution rates (mg. cm$^{-2}$.day$^{-1}$) (Table II). After overnight fasting, monkeys were restrained in chairs and chest areas clipped to remove hair. Skin surface was then wiped clean with the isopropyl alcohol solution. A four square centimeter portion of the pad was then cut and glued on the center of the adhesive backing. The delivery system containing these two components was then applied on the chest area, pressing gently for proper adhesion. After 24 hours, the delivery system was carefully removed from monkeys, and the pad separated from the adhesive backing. Initial albuterol content was estimated from the weight of the pad, the content uniformity, and percent loading. The residual content in the pad was determined by extracting the pad with acetone. The extracts were analyzed by an HPLC method to determine residual albuterol in the pad. From the initial and final albuterol content in the pad, its loss and hence the in vitro release rates (mg./cm$^2$ .day) were calculated. These are listed in Table III.

Serum concentration time profiles after intravenous administration : Four female rhesus monkeys, #388, #391, #423, and #430, were used in a cross over design for IV and pad bioavailability experiments. From each monkey, 7 ml of blood sample was removed on the previous day of the experiment, centrifuged to separate serum, and stored at -20°C till the analysis. This serum sample was used to make blanks and standards. Following this. monkeys were fasted overnight, restrained and settled in chairs before the intravenous injection. Albuterol was dissolved in normal saline (0.9% sodium chloride) solution in such a way that the dose was 50 mcg/kg for 1ml/kg injection. Albuterol solutions were then injected into the saphenous vein. Five milliliter blood samples were removed at 0.00, 0.08, 0.17, 0.33, 0.75, 1.00, 1.5, 2.00, 3.00, 4.00, and 5.00 hours following 50 mcg/kg injection. The serum was separated from blood via centrifugation and stored at -20°C till analysis.

Serum concentration time profiles after transdermal pad administration . After overnight fasting, on the day of the experiment, monkeys were restrained in chairs and the chest areas were clipped to remove hair, avoiding any injury to the skin tissue. Skin surface was wiped with isopropyl alcohol swab and a 4 square centimeter portion of the pad was then applied to the chest area for 24 hours and was removed as described previously. Following the pad application, blood samples were removed at 0.00, 0.5, 1.00, 1.50, 3.00, 5.00, 7.00, 12.00, 24.00, 31.00, and 48.00 hours, centrifuged to separate serum and stored at -20°C till the analysis.

Analysis of serum samples . Serum albuterol and internal standard bamethan sulphate were extracted into chloroform to remove polar interfering substances and reextracted in to the aqueous phase to eliminate nonpolar materials. The aqueous extracts were analyzed by an HPLC-Fluorescence method. Pharmacokinetic parameters were estimated by conventional pharmacokinetic methods well known to those of ordinary skill in the art.

Results : Following IV injection of 50 mcg/kg dose of albuterol, the initial half lives obtained were 6.0 minutes. Similarly, the terminal half lifes was 135.6 minutes.

The volume of the central compartment was 362.46 ml/kg after 50 mcg/kg doses. Serum drug concentrations observed after 50 mcg/kg I.V. dosing are listed in table IV in ng/ml.

The serum concentration time data and pharmacokinetic parameters of albuterol obtained after transdermal pad application are shown in tables V and VI. The time course of transdermal albuterol showed a steady incline up to 12 hours ($t_{ss}$) following a dual layer pad application. Thereafter, the steady state concentrations were maintained till the pad was removed at 24 hours. In monkeys 423 and 430, however, after application of a dual layer pad, albuterol concentrations were higher at 24 hours than at 12 hours. Since blood samples were not withdrawn between these time intervals, it was difficult to establish the time to achieve steady-state ($t_{ss}$). In all the monkeys, however, drug concentrations declined rapidly after the pad was removed, with no measurable concentration remaining at 48 hours.

Comparison of in vitro release rates obtained from the pad residue analysis and in vivo absorption rates $K_0$ calculated from pharmacokinetic parameters is given in table VII.

Comparison between in vitro - in vivo parameters of albuterol pads is given in table VIII. Hypothetical serum concentrations were calculated from $K_0$ and clearance (C1) in monkeys and then extrapolated to a 70 kg human (table IX).

Dose versus area under the curve relationships following intravenous and transdermal application of albuterol is give in table X.

TABLE I

| HAIRLESS MOUSE SKIN PERMEATION RATE CONSTANTS. $(mg.cm^{-2}.day^{-1})$ | | |
|---|---|---|
| Dual | Layer | Pad |
| 0.38, 0.43, 0.47, | 0.49, 0.41, 0.50. | 0.40 0.50 |
| **Mean: 0.45** | | |
| S.D.: 0.05 | | |

TABLE II

| PAD DISSOLUTION RATE CONSTANTS $(mg.cm^{-2}.day^{-1})$ | | |
|---|---|---|
| Dual | Layer | Pad |
| 2.52, | 2.09, | 2.49 |
| **Mean: 2.37** | | |
| S.D.: 0.25 | | |

TABLE III

| Residual pad analysis after application to monkey skin. | |
|---|---|
| Parameter | Dual Layer / Pad |
| Initial Amount $(mg.cm^{-2})$ | 5.56(1.30)[1] |
| Residual Amount $(mg.cm^{-2})$ | 3.60(0.82) |
| In vitro release rate $(mg.cm^{-2}.day^{-1})$ | 1.96(0.46) |

1. Number in parentheses is standard deviation of the mean.

TABLE IV

| Serum concentration time data following intravenous injection of 50 mcg/kg albuterol in rhesus monkeys. (ng/ml) | | | | | |
|---|---|---|---|---|---|
| Monkey #388 | Monkey #391 | Monkey #423 | Monkey #430 | Average | S.D. |
| 0.00 | 0.00 | 0.00 | 0.00 | | |
| 102.00 | 73.60 | 84.30 | 73.00 | 57.72 | 38.83 |
| 40.70 | 56.90 | 46.80 | 45.00 | 47.35 | 8.40 |
| 23.70 | 39.70 | 29.10 | 31.60 | 31.02 | 6.65 |
| 13.20 | 17.90 | 19.60 | 16.60 | 16.82 | 2.71 |
| 10.60 | 18.70 | 14.30 | 13.80 | 14.35 | 3.33 |
| 8.00 | 12.50 | 14.60 | 11.20 | 11.58 | 2.76 |
| 8.74 | 11.50 | 12.70 | 9.66 | 10.65 | 1.78 |
| 5.63 | 7.55 | 12.30 | 8.32 | 8.45 | 2.80 |
| 5.43 | 6.33 | 7.84 | 6.04 | 6.41 | 1.02 |
| 6.82 | 4.53 | 7.59 | 4.13 | 5.77 | 1.70 |

TABLE V

| Serum concentration time data following transdermal application of albuterol dual layer paid in rhesus monkeys. | | | | |
|---|---|---|---|---|
| Monkey #388 | Monkey #391 | Monkey #423 | Average | S.D. |
| 0.000 | 0.287 | 0.000 | | |
| 0.000 | 0.000 | 0.000 | | |
| 0.000 | 0.000 | 0.000 | | |
| 0.000 | 0.000 | 0.779 | 0.26 | 0.45 |
| 1.270 | 2.270 | 8.790 | 4.11 | 4.08 |
| 24.200 | 14.900 | 25.100 | 21.40 | 5.64 |
| 44.700 | 22.200 | 33.200 | 33.36 | 15.91 |
| 58.500 | 42.900 | 53.400 | 51.60 | 7.95 |
| 59.300 | 68.100 | 65.600 | 64.33 | 4.53 |
| 10.900 | 26.900 | 33.300 | 23.70 | 11.54 |
| 0.000 | 0.000 | 0.000 | 0.00 | 0.00 |

TABLE VI

| Mean and standard deviation of pharmacokinetic parameters obtained after transdermal application of albuterol dual layer pads in rhesus monkeys (n = 4) | | |
|---|---|---|
| Parameter | Mean | SD |
| Weight | 5.33 | 0.20 |
| AUC (ng.ml$^{-1}$.hrs) | 1500.80 | 191.47 |
| Css (12-24 hrs) (ng.ml$^{-1}$) | 57.90 | 2.16 |
| K$_o$ (mg.day$^{-1}$.cm$^{-2}$) | 1.09 | 0.23 |
| AUC = Area Under Curve | | |
| C$_{ss}$ = Serum Concentration at Steady State | | |
| Cl = Clearance Rate | | |
| K$_o$ = In Vivo Absorbtion Rate Constant | | |

TABLE VII

Comparison of in vitro-in vivo parameters obtained following pad residue analysis and in vivo absorption rates of albuterol dual layer pads in monkeys.

| | Subject | In vitro release rate (mg.cm$^{-2}$.day$^{-1}$) | K$_o$ (mg.cm$^{-2}$.day$^{-1}$) |
|---|---|---|---|
| Dual Layer | (Monkey #423 | 1.04 | 0.92 |
| | ( | | |
| | (Monkey #430 | 0.96 | 1.03 |

TABLE VIII

| Comparison of in vitro parameters of albuterol pads. | | |
|---|---|---|
| | Dual Layer | |
| Parameter (mg.cm$^{-2}$.day$^{-1}$) | Mean | S.D. |
| Dissolution rate constant. | 2.37 | 0.25 |
| Hairless mouse skin permeation rate constant. | 0.45 | 0.05 |
| Monkey skin permeation rates constant | 1.96 | 0.46 |

TABLE IX

| Hypothetical serum concentrations after application of albuterol pads in a 70 KG human. | |
| --- | --- |
| Pad Size (cm$^2$) | Serum Concentration (ng.ml$^{-1}$) Dual Layer |
| 4.0 | 3-5 |
| 8.0 | 6-10 |
| 16.0 | 12-20 |

TABLE X

| Dose versus area under the curve comparison following intraveous and transdermal application of albuterol in rhesus monkeys. | | | | |
| --- | --- | --- | --- | --- |
| Monkey Identification | | | | |
| Parameter | #388 | #391 | #423 | #430 |
| Intravenous dose (mg.Kg$^{-1}$) | 0.05 | 0.05 | 0.05 | 0.05 |
| Transdermal dose (mg.Kg$^{-1}$) | 0.38 | 0.39 | 0.77 | 0.72 |
| AUC Area Under the Curve after intravenous administration (ng.ml$^{-1}$ hr) | 70.69 | 85.72 | 106.39 | 73.96 |
| AUC Area Under the Curve after transdermal administration (ng.ml$^{-1}$ hr) | 711.51 | 777.2 | 1534.52 | 1259.29 |

EXAMPLE 2

A double layer formulation can be made, having two discrete, separate albuterol and dodecanol layers.

100 g of albuterol layer mixture was prepared with 74.00 g of Dow X7-3058 silastomerTM, 20 g albuterol, 5.0 g glycerol 1.0 g hexanol and catalyst.

100 g of dodecanol layer was prepared with 90.0 g of Dow X7-3058 silastomerTM, 10 g dodecanol and catalyst.

For each layer, catalyst was mixed with Silastomer in a clean mortar according to methods well known to those of ordinary skill in the art.

In a clean mortar a fine paste of albuterol, hexanol and glycerol was made. One portion of the first silastomer-catalyst mixture was mixed thoroughly with the albuterol paste. Following this, the remaining two portions of the first silastomer-catalyst mixture are mixed one at a time with the albuterol paste.The dodecanol layer was prepared by mixing the dodecanol with the other silastomer-catalyst mixture.

The resulting masses of the albuterol and dodecanol layers were separately processed as follows. Each mass was passed through a triple roller mill to obtain a homogeneous mixture. Each mixture was then placed between two sheets of mylar plastic film and passed through two aluminum rollers of a film casting apparatus. Each spread was then cured in an oven at 100° C for 10 minutes.

The cured films of albuterol and dodecanol layers were cut into pieces, each measuring four square centimeters. The dodecanol layer was placed on top of the albuterol layer. The side of the dodecanol layer not in contact with the albuterol layer was glued to a four square centimeter patch of aluminum foil. The surface of the foil not in contact with the dodecanol layer was then adhered to a patch of adhesive-lined foam.

EXAMPLE 3

100 g of albuterol pad formulation was prepared with 71.90 g Dow X7 - 3058 Silastomer™, 16 g albuterol, 10 g n-dodecanol, 1.75 g glycerol, 0.35 g hexanol and X7 - 3075 catalyst, available from Dow Corning.

In a clean mortar, catalyst was mixed with silastomer in a geometric dilution according to methods well known to those of ordinary skill in the art.

In a clean mortar, a fine paste of albuterol, dodecanol, hexanol and glycerol was made. One portion of the silastomer-catalyst blend was mixed thoroughly with the albuterol paste. Following this, the remaining two portions of the silastomer-catalyst blend were mixed one at a time with the albuterol paste.

The resulting mass was passed through a triple roller mill to obtain a homogeneous mixture. The mixture was then placed between two sheets of mylar plastic film and passed through twin aluminum rollers of a film casting apparatus well known to those of ordinary skill in the art. The thickness of the spread can be adjusted by manipulating the gap between the two rollers. The resulting spread was then cured in an oven at 100° C for ten minutes.

The cured film was cut into four square centimeter pieces and each piece was glued onto a 4 square cm patch of aluminum foil. The surface of the foil not in contact with the pad was then adhered onto a patch of adhesive-lined foam.

Any number of such integrated albuterol/dodecanol layers can be made and laminated into a patch simply by placing layers on top of one another by the method of Example 2.

Therapeutically active agents which produce a systemic activity and which are deliverable by the present invention are, for instance, and without limitation, anti-infectives for example lomefloxacin or pentamidine, antibiotics for example metronidizole, hormones, antipyretics, antidiabetics, coronary dilation agents, glycosides, spasmolytics, antihypertensives for example verapamil or its enantiomers or betaxolol, psychoactive agents for example zolpidem, cycloserin or milacemide, corticosteroids, analgesics, contraceptives, nonsteroidal anti-inflammatory drugs for example oxaprozen, anticholinergics, sympatholytics, sympathomimetics, vasodilatatory agents, anticoagulants, antiarrhythmics for example disopyramide or disobutamide, or prostaglandins having various pharmacologic activities for example misoprostol or enisoprost.

While the invention has been described and illustrated with reference to certain prepared embodiments thereof, those skilled in the art will appreciate that various changes, modifications and substitutions can be made therein without departing from the spirit and scope of the invention. Likewise, the specific pharmacological responses observed may vary according to and depending upon the particular active compounds selected. It is intended therefore that the invention be limited only by the scope of the claims which follow and that such claims be interpreted as broadly as is reasonable.

## Claims

1. A laminated multi-layer patch for the transdermal administration of a drug comprising:
   a. at least two elastomeric matrices of predetermined thickness and area;
   b. an active drug ingredient dispersed throughout at least one of the matrices; and
   c. a diffusion enhancer dispersed throughout at least one of the matrices.

2. The patch as claimed in claim 1, additionally comprising a plasticizer.

3. The patch as claimed in claim 2, and which the plasticizer comprises gylcerol.

4. The patch as claimed in claim 1, additionally comprising a solubilizer.

5. The patch as claimed in claim 4, in which said solubilizer comprises n-hexanol.

6. The patch as claimed in claim 1, additionally comprising a curing catalyst for said elastomer.

7. The patch as claimed in claim no. 1, in which said diffusion enhancer is a normal hydrocarbon alcohol.

8. The patch as claimed in claim no. 7, in which said alcohol is n-dodecanol.

9. The patch as claimed in claim 1, in which said active drug ingredient is a $\beta_2$ adrenergic-receptor agonist.

10. The patch as claimed in claim 1, in which said active drug ingredient is a bronchodilator.

11. The patch as claimed in claim 1, in which said active drug ingredient is albuterol.

12. A laminated multi-layer patch for the transdermal administration of a drug comprising:
    a. at least two silicone elastomeric matrices of predetermined thickness and area;
    b. a pharmacologically sufficient amount of albuterol, dispersed throughout at least one of said matrices; and
    c. n-dodecanol as a diffusion enhancer for said albuterol, dispersed throughout at least one of said matrices.

13. The patch as claimed in claim 12, in which said elastomer is present in an amount ranging from about 45 to about 95 percent, weight to weight.

14. The patch as claimed in claim 13, in which said elastomer is present in an amount ranging from about 65 to about 90 percent, weight to weight.

15. The patch as claimed in claim 12, in which said albuterol is present in an amount ranging from about 2 to about 30 percent, weight to weight.

16. The patch as claimed in claim 15, in which said albuterol is present in an amount ranging from about 5 to about 25 percent, weight to weight.

17. The patch as claimed in claim 16, in which said albuterol is present in an amount ranging from about 6 to about 16 percent, weight to weight.

18. The patch as claimed in claim 17, in which said albuterol is present in the amount of about 10 percent, weight to weight.

19. The patch as claimed in claim 12, in which said n-dodecanol is present in an amount ranging from about 2 to about 30 percent, weight to weight.

20. The patch as claimed in claim 19, in which said n-dodecanol is present in an amount ranging from about 3 to about 7 percent, weight to weight.

21. The patch as claimed in claim 20, in which said n-dodecanol is present in the amount of about 5 percent, weight to weight.

22. The patch as claimed in claim 12, which is capable of delivering said active drug ingredient at a release rate ranging from about 0.2 to about 2 milligrams per square centimeter of patch area per day.

23. The patch as claimed in claim 22, which is capable of delivering said active drug ingredient at a release rate ranging from about 0.3 to 0.85 milligram per square centimeter of patch area per day.

24. Use of the patch of claim 1 for preparing a medicament for treating bronchial constriction in a mammal in need thereof.

25. Use of the patch of claim 1 for preparing a medicament for delaying premature uterine contractions in a pregnant mammal in need thereof.

26. Use of the patch of claim 1 for preparing a medicament for treating urticaria in a mammal in need thereof.

MASS TRANSFER PROFILING
OF ALBUTEROL
USING THE FRANZ CELLS AND WHOLE HAIRLESS MOUSE

Figure 1

## MASS TRANSFER PROFILING
## OF ALBUTEROL
## USING THE FRANZ CELLS AND WHOLE HAIRLESS MOUSE

Legend

■ DODECANOL
□ NONYL ALCOHOL
● HEXANOL
○ n-PROPANOL
△ DECANOL
X EtOH
▽ NEAT II
+ NEAT I

Figure 2